# EUROPEAN PATENT SPECIFICATION

(11) **EP 3 247 327 B1**
(45) Date of publication and mention of the grant of the patent: **07.08.2019**
(21) Application number: 16701276.4
(22) Date of filing: 20.01.2016
(51) Int. Cl.: A61K 9/00, B29C 67/00

(54) **DRUG DELIVERY SYSTEM COMPRISING A NON-STEROIDAL ANTI-INFLAMMATORY (NSAID) AND A PROGESTOGENIC COMPOUND AND METHODS FOR MANUFACTURING**
ARZNEIMITTELSYSTEM MIT EINER NICHTSTEROIDEN ENTZÜNDUNGSHEMMENDEN (NSAID) UND PROGESTOGENEN VERBINDUNG UND VERFAHREN ZUR HERSTELLUNG
SYSTÈME D'ADMINISTRATION DE MÉDICAMENT COMPRENANT UN ANTI-INFLAMMATOIRE NON STÉROÏDIEN (AINS) ET UN COMPOSÉ PROGESTATIF, ET PROCÉDÉS DE FABRICATION

(30) Priority: 21.01.2015 US 201562106073 P
(43) Date of publication of application: 29.11.2017
(73) Proprietor: Bayer Oy, 20210 Turku (FI)
(72) Inventor: JUKARAINEN, Harri, 21620 Kuusisto (FI); PIHLAJA, Jyrki, 21530 Paimio (FI); HOLMBERG,Svante, 20900 Turku (FI); VALO, Tuula, 20740 Turku (FI); HONKA, Anu-Liisa, 20100 Turku (FI); HOLLAENDER, Jenny, 21600 Parainen (FI)
(74) Representative: BIP Patents
(86) International application number: PCT/EP2016/051135
(87) International publication number: WO 2016/116502

(56) References cited:
- WO-A1-2006/121969
- WO-A1-2010/000943
- WO-A1-2010/000943
- WO-A1-2014/055850
- JENNY HOLLAENDER: "Thesis, 3D printing of medical device prototypes", THESIS, DEPARTMENT OF BIOSCIENCES, ABO AKADEMI UNIVERSITY, FI, 1 January 2014 (2014-01-01), pages 1-124, XP009506621,

## Description

The present invention is related to drug delivery system comprising non-steroidal anti-inflammatory (NSAID) and a progestogenic compound and methods for manufacturing of a drug delivery system comprising a progestogenic and an anti-inflammatory active compound, under the condition of 3 D printing and a device constructed with the process.

Implantable PBDSs for contraceptives have been studied since the 1960s. The implantable PBDS for contraceptives has been manufactured as rods (implants), intrauterine system (IUS) and intra vaginal ring (IVR). There are a limited number of polymers that can be used for in-vivo drug delivery systems such as as IUSs or IVRs, since they have to be non-swellable and non-biodegradable or the degradation rate has to be very slow. The marketed IUSs and IVRs, consists of non-degradable polymers such as polydimethylsiloxane (PDMS) or EVA. The device backbone in the IUS that is currently on the market, Mirena®, is made of polyethyene with a drug-delivery cylinder wrapped around it. The IVRs, Progering® and Nuvaring®, are made of silicone rubber/PDMS and EVA, respectively. The devices are manufactured by extrusion and injection molding.

To improve theses product further and to reduce side effects such as initial bleeding and spotting a lot of research is ongoing where the contraceptive active drug is combined with additional drugs such as NSAID's. The purpose of the combinations is to gain a multiple effect by using a single product, such as to prevent sexually transmitted infections in additional with the contraceptive effect of a DDS. A non-hormonal copper IUS containing indomethacin is another example of DDS with a multipurpose application. The indomethacin is incorporated to reduce the side effects of the IUS (CN 1 931 113 A, US 2004/247674 A1 and WO 2004/096151 A2).

Intrauterine delivery systems comprising two active compounds are disclosed in WO 2010/000943 A1 and EP 2 905 014 A1. However, in these intrauterine systems both active compounds are located in separate (silicon) reservoirs which are mounted on the drug free T-frame. The size of the reservoir limits the amount of drug which can be contained and delivered. In particular in cases where a higher drug amount is needed, these intrauterine systems (IUS) are not suitable. It was therefore one object of the present invention to provide a drug delivery system capable to hold larger amounts of one active ingredient. A method for making a mammalian subcutaneous medical implant for controlled releasing self-contained drugs via 3D printing is disclosed in WO 2014/055850. Medical device may have two reservoir core layers containing two different drugs and may be possibly coated. The drug-containing matrix is athermoplastic EVA polymer and may be biodegradable or non-biodegradable.

There is also a need to improve the methods for the manufacturing of such devices, especially for these drug delivery systems, wherein one drug is contained in the frame material, in an IUS wherein the progestogenic compound contained in the silicon capsule is Levonorgestrel and wherein the anti-inflammatory active compound which is contained in the frame material is indomethacin.

Preferably the drug delivery system is an intrauterine System (IUS), in particular an intrauterine system with a frame in the form of a T (T-frame). However, the manufacturing method is likewise applicable to other drug delivery systems such as drug containing intravaginal rings (IVR) or drug containing implants.

Polymer based drug delivery systems (DDS) are known for decades. The used polymers can be divided into two main groups, (i) biodegradable and (ii) non-degradable polymers. Biodegradable polymeric DDS are usually matrix systems, whereas non- degradable polymers can be as reservoir or matrix systems. The biodegradable polymers were developed for the biomedical and the pharmaceutical field with the aim that they would degrade in the body in a controlled rate into non-toxic products that could be eliminated through natural ways.

One common biodegradable polymer used in DDS is polycaprolactone (PCL). PCL have high permeability to many drugs which makes it suitable for long-term DDS and has excellent biocompatibility.

One of the most common non-degradable polymers that has been used for decades in the biomedical and pharmaceutical field is poly(ethylene-co-vinyl acetate) (EVA or PEVA). EVA is a chemically inert, biocompatible and an insoluble polymer.

In the context of the current invention no-degradable polymers are preferred as in addition to the drug contained in the T-frame a silicon capsule containing the progestin is mounted on the frame. Thus the silicon capsule would lose it "anchor" if the frame material is biodegraded.

An intrauterine delivery system comprising Indomethacin and Levonorgestrel,characterized in that the Indomethacin is contained in the frame material and that levonorgestrel is contained in a silicon based reservoir attached to the frame, wherein the frame consist of ethinylvinylacetate copolymer with 16 % vinylacetate content (EVA 5).

A method for manufacturing of a frame of an intrauterine delivery system comprising Indomethacin and Levonorgestrel, wherein the Indomethacin is contained in the frame and levonorgestrel is contained in a silicon based reservoir attached to the frame, characterized in, that a mixture of a ethinylvinylacetate copolymer with 16 % vinylacetate content (EVA 5) and the Indomethacin are treated under the condition of 3-D printing methods.

In the context of the current invention the following abbreviations are used:
- 3D - Three- dimensional
- ABS - Acrylonitrile butadiene styrene
- API - Active pharmaceutical ingredient
- ATR-IR - Attenuated total reflection infrared spectroscopy
- CAD - Computer-aided design
- DDD - Drug delivery devices
- DDS - Drug delivery systems
- DSC - Differential scanning calorimetry
- EVA - Poly(ethylene-co-vinyl acetate)
- FDM - Fused Deposition Modelling
- FFF - Fused filament fabrication
- HME - Hot-met extrusion
- ID - Inner Diameter
- IND - Indomethacin
- IUD - Intrauterine device
- IUS - Intrauterine system
- IVR - Intra vaginal ring
- LDPE - Low density polyethylene
- MRI - Magnetic resonance imaging
- NSAID - Non-steroidal anti-inflammatory drug
- PCL - Polycaprolactone
- PEVA - Poly(ethylene-co-vinyl acetate)
- PLA - Polylactic acid
- OD - Outer Diameter
- SD - Standard deviation
- SEM - Scanning electron microscopy
- VA - Vinyl acetate
- XRD - X-ray diffraction

One object of this invention is to explore the potential of 3D printing in fabrication of drug delivery devices (DDD) of two polymers, polycaprolactone (PCL) and ethylene-vinyl acetate (EVA), with the main focus on printing of drug-containing intrauterine systems (IUS) and the the anti-inflammatory active compound is contained in the frame material. This object contains manufacturing drug-containing T-intrauterine systems (IUS) with the drug incorporated within the entire backbone of the medical device by using 3D printing technique, based on fused deposition modelling (FDM™). Indomethacin was used to prepare drug-loaded poly-caprolactone (PCL)-based filaments with different drug contents up to 40 wt%, in particular with 5%, 15% or 30% wt% of Indomethacin. As a further polymer EVA and EVA mixtures with VA are suitable.

This invention proves that it is possible to print drug-loaded types of PCL and certain grades of EVA with the 3D printing technique. The printing process, though, is a complex interplay between many variables and parameters and the process thus needs optimization for each new feedstock. The drug release from the printed devices depended on the geometry of the devices, the matrix polymer and the degree of the crystallinity of the incorporated drug. Further investigations of the printed T-frames regarding mechanical strength, the stability of the drug in the polymer and the effect on different drug loadings and additives must be conducted in order to produce products according to the current invention.

In the context of this invention the printability of PCL as well as of different grades of ethylene vinyl acetate (EVA) copolymers (EVA 1 to EVA 12) as new feedstock material for fused-deposition modeling (FDM™)-based 3D printing technology in fabrication of custom-made T-shaped intrauterine systems (IUS) and subcutaneous rods (SR) has been investigated.

It was a further object of the invention to select an EVA grade with the optimal properties, namely vinyl acetate content, melting index, flexural modulus, for 3D printing of T-frames with the drug incorporated within the entire matrix of the medical devices. As it turned out EVA 5 (with wt % VA) is preferred in the context of the current invention.

The feedstock filaments were fabricated by hot-melt extrusion (HME) below the melting point of the drug substance and the IUS and SRs were successfully printed at the temperature above the melting point of the drug.

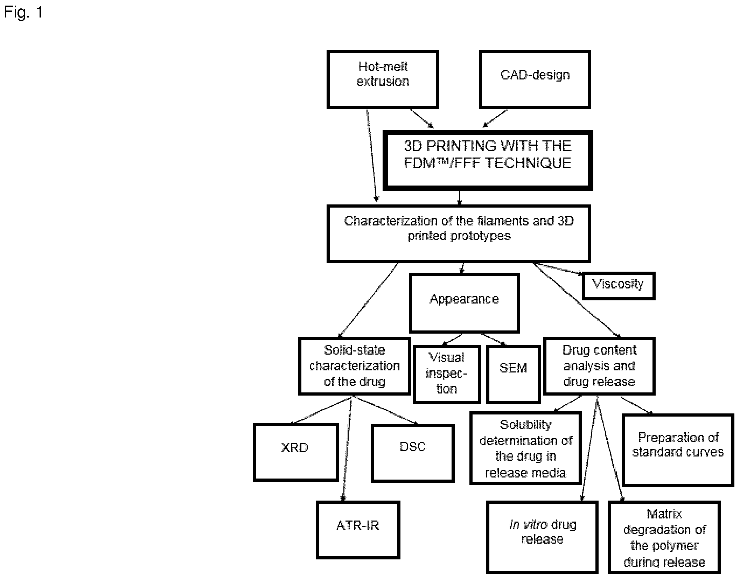

The manufacturing process of the IUS or IVR and the following characterization methods of the properties of the devices are presented in Figure 1.

The process for manufacturing of the drug containing IUS or ring consisted of the following steps: (i) filament manufacturing by hot melt extrusion, (ii) CAD designing of the T-frames and (iii) printing of the samples with a 3D printer. For the current examples a desktop 3D printer was used. However, in industrial production other 3D printers are likewise suitable.

### Example 1 (Starting materials)

As drug indomethacin was used. Indomethacin can appear in different polymorphic forms, which are differently bioavailable. In the current examples the stable form γ-indomethacin was used. As it was proven with respective analytical methods (i.a. X-ray diffraction) the polymorph form does not change during filament preparation and printing. Other NSAID's such as meloxicam, piroxicam, naproxen, celecoxib, diclofenac, tenoxicam, nimesulide, lornoxicam and indomethacin are likewise suitable, of which indomethacin is particularly preferred.

Commercial available polymers (PCL and different EVA polymers) had been furthermore used wherein EVA5 is preferred.

### Example 2 (Filament Preparation)

The hot melt extrusion was done with a HAAKE miniCTW micro-conical twin-screw extruder (Thermo Fisher Scientific, Karlsruhe, Germany). The extruder is a small-scale conical twin screw extruder with co- and counter-rotating screws. The load of the extruder is 7 cm³.

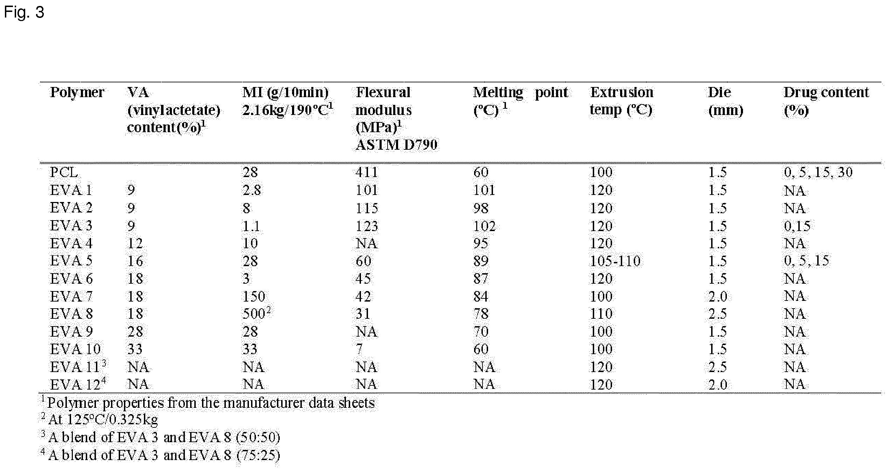

To begin the hot melt extrusion process, the extruder temperature has to be adjusted first. The applied extrusion temperature was about 15 - 40 °C above the melting point of the polymer, depending on the properties of the respective polymer (Figure 3). All selected extrusion temperatures were below the melting point of indomethacin. After the extruder had reached the target temperature the screw rotation was turned on. The rotation speed for the melting and blending process was set to 30 rpm. The API and the polymer were separately weighed into small plastic bags. First, about 1/5 of the polymer were fed into the extruder and when it had melted, subsequently, micronized indomethacin and the polymer were added into the extruder hopper. When feeding and blending, the extruder was run in a circulation mode, and therefore it was possible to feed the materials separately. The extruder had a manual feed mechanism. The materials were fed into the barrel, when a piston was pressed down in the hopper. The torques in the extrusions varied from 0.20-1.45 Nm.

The residence time was 10 minutes. Residence time in the barrel was defined as the time from which all material was fed into barrel and the torque had stabilized, until the die (template; matrix) at the end of the barrel was opened. After blending for 10 minutes the rotation speed was set to 10 rpm and the drug-polymer mixture was extruded as filament through a die that was located at the end of the barrel. The used dies were 1.5-2.5 mm in diameter, depending on the swelling properties of the polymers. The filament diameter was chosen according to the recommendation of the manufacturer of the printer. If other printers are used the diameter has to be adjusted accordingly. Here the filament diameter for the printing was 1.75 mm ± 0.05 mm.

The diameter of the extruded filament was controlled on-line with a laser diameter measurement device (HAAKE, Karlsruhe, Germany) equipped with a data display (Zumbach USYS, Orpund, Switzerland). The equipment was placed right after the extruder to directly monitor the diameter of the extruded filament. A conveyer belt (Thermo Scientific, Karlsruhe, Germany) was placed after the diameter monitoring equipment to slowly cool down the coming filament as well as to adjust the filament diameter to the desired range by changing the speed of the belt. When the extruded filaments had cooled down, their diameter was measured again.

### Example 3 (Printing)

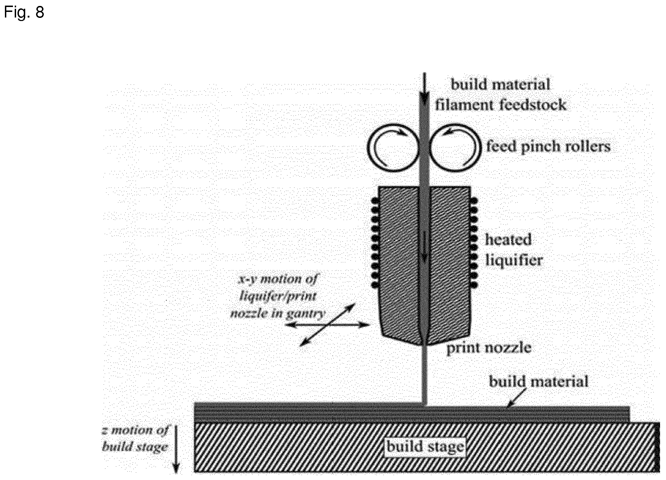

3D printing was performed with a MakerBot Replicator 2 (USA) desktop printer, which uses the fused filament fabrication technique (FFF) for 3D printing. A typical FDM/FFF extruder is shown in Figure 8. FFF is a solid freeform fabrication technique based on the fused deposition modelling, FDM™, patented by Stratasys. The printer original feedstock materials are PLA and PCL. The printing process starts usually with loading the filament into the printer and importing the 3D CAD model into the printer software. When the filament is loaded and the file imported the printing process begins.

The filament loading process started with heating of the liquefier and nozzle to temperatures well above the melting point of the polymer, about 60 °C - 115 °C above melting point, depending on the properties of the polymer. When the set temperature for the loading was reached, the filament was fed into the liquefier via pinch rollers until melted polymer got extruded from the nozzle. The purpose of the extrusion was to empty the liquefier and nozzle from previous filament residues and to check that the flow of the extruded material was good enough for printing. The default nozzle size in MakerBot Replicator 2 was 0.4 mm.

When the filament was successfully loaded and the flow was appropriate, the printing process continued by importing the 3D CAD model into the MakerWare software. Other files that the MakerWare software supports are ".STL", ".obj" or ".thing" files. The software has a slicing tool, called MakerBot slicer, which translates the 3D CAD model into a code for the printer by slicing it into thin horizontal layers.

The selection of the appropriate printing material plays an important role in order to proceed with the successful printing. The suitable material for the FFF process has to be in the form of filament with the right diameter, flexural modulus and strength and flow properties (Comb et al., 1994)¹.

Besides the material properties, the printers hard- and software process parameters as well as the T-frame geometry are crucial for a successful printing and a good quality of the created T-frames. In the a.m. examples the main focus has been pointed at the material properties, such as filament stiffness, viscosity and drug-loading. In addition, some hardware properties, such as build plate and loading system and software properties, such as printing temperatures and speeds, are discussed.
¹ COMB, J.W., PRIEDEMAN, W.R. and TURLEY, P.W., 1994. FDM technology process improvements. in Marcus, H.L, BEAMAN, J.J., BARLOW, J.W., BOURELL, D.L. and CRAWFORD, R.H. (Eds), Proceedings of the Solid Freeform Fabrication Symposium, Vol.5, 1994, The University of Texas at Austin, Austin TX, pp. 42-49

With both PCL and EVA and both the drug-free and the drug-loaded filaments, the challenges with the loading and printing process were, (i) the filament diameter, (ii) build plate adhesion and (iii) geometry of the drug delivery system.

The diameter of the extruded filaments varied due to manufacturing challenges of the HME process. It led to the loading problems of the filament during 3D printing. Briefly, the filament is fed into the liquefier of the 3D printer via pinch rollers, and a stepper motor is connected to one of the rollers providing energy to move the filament down the system. The printer used in this study had counter-rotating steel rollers with diameters of about 5 and 10 mm. The smaller roller had a smooth surface and the bigger roller which is connected to the motor, had a surface with a grooved texture. Too thick filaments could not be fed, because the liquefier diameter was only a little bigger than the desired dimensions of the filaments (1.75+0.05 mm). Filaments thinner than 1.70 mm could not be fed, because of unsufficient friction between the rollers, leading to too low pressure on the filament with slipping as a result. By changing the properties of the rollers (their dimensions and materials), the limits of the desired diameters of the filaments could be wider. Comb et al. (1993)² has studied the required drive traction of feeding systems, with different roller sizes and surface materials, to load the filament into the liquefier without slipping. Drive traction is the force provided by the feeding system to load the filament into the liquefier. It was reported that smaller (1/2") elastomeric wheels increases the traction force, due to their higher coefficient of friction, and are therefore better able to conform to variations of the filament, than the bigger wheels.

The printer has an acrylic build plate in the default setup, but neither PCL nor EVA did adhere to it properly. Therefore, PCL frames were built on Kapton polyimide tape. EVA did not adhere to the polyimide tape and after testing different materials, e.g. glass, painter tape, different plastics, aluminum, the EVA frames were printed on LDPE films, because it had the best attaching properties of all tested surfaces.

All IUS frames were printed with rafts, because unsupported frames wrapped during printing on the build plate. The rafts adhered better to the build plate as they were of larger attaching-to-the plate area than frames alone. In addition, the adhesion problem of the printed frames was partly due to the geometry of the frames and partly due to surface characteristics of the build plate as well as ambient conditions such as the environment temperature. The heat capacity and the thermal conductivity of the material determines the viable process temperatures. During printing below the desired temperature range, bonding
² COMB, J.W. and PRIEDEMAN, W.R., 1993. Control parameters and material selection criteria for rapid prototyping systems, in Marcus, H.L, BEAMAN, J.J., BARLOW, J.W., BOURELL, D.L. and CRAWFORD, R.H. (Eds), Proceedings of the Solid Freeform Fabrication Symposium, Vol.4, 1993, The University of Texas at Austin, Austin TX, pp. 86-91
or adhesion to the build plate, adjacent roads and layers are poor. A printer with an adjustable envelope temperature and a heated or a vacuum build platform could have decreased the adhesion problems to the build plate.

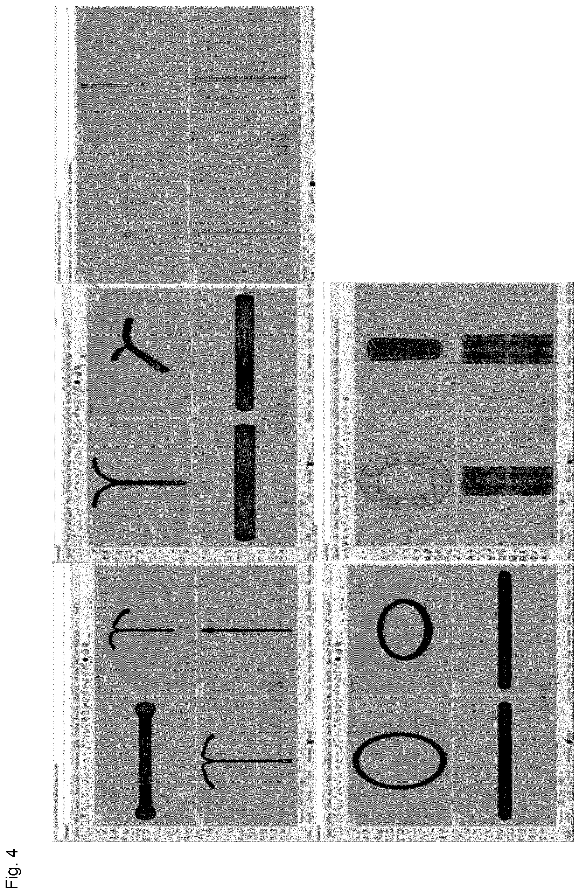

Two different IUS structures have been printed (IUS1 and IUS2) as well as a ring and a rod structure have been printed. The different structures are shown in Figure 4.

The printed IUS 1 needed a support structure to be built on, due to the geometry of the T-frame. As the printer had only a single extruder, the support structure was printed with the same pure polymer, without any drug inside. The support structure was then manually cut off from the T-frame after cooling down. The removing of the supports structure affects the frame surface (Agarwala et al. 1996)³.

Frames that can be built without a need for any support structures have better surface finish than those built with additional supportive elements. Some of the impact of the support structure on the final frame surface, can be decreased by using a dual-extruder printer. With such a printer, the support structure can be built from an alternate build material, which forms weaker interfaces with the actual build material, and can, therefore, be more easily removed. The frame *Sleeve* could not be printed with any of the tested drug-free or drug-loaded polymers. The geometry of this tube was: OD 2.9 mm, ID 1.5 mm and length 5 mm.

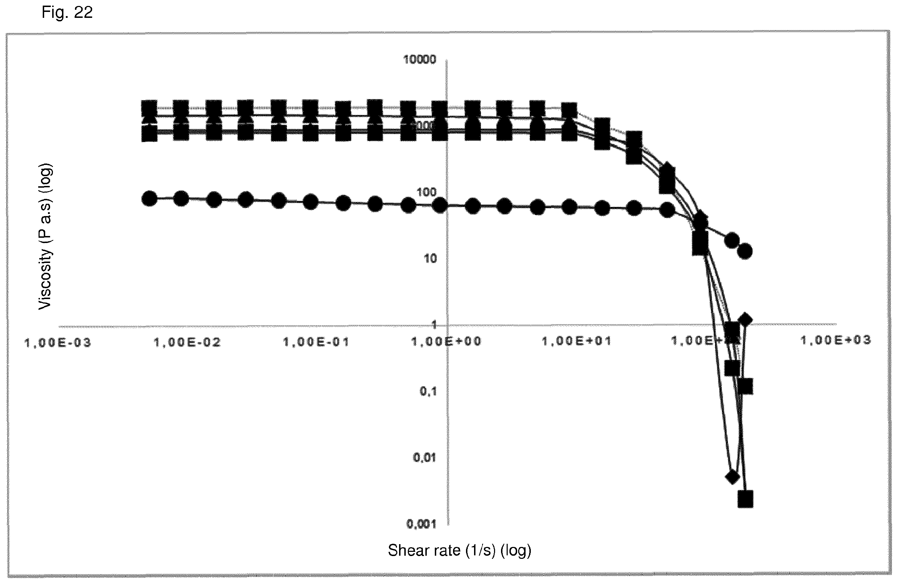

PCL is one of the original feedstock materials for the printer. The default printing speed for PCL is 45 mm/s. The maximum printing speed for a material depends on the process parameters such as the printed road width and height, printing temperature and nozzle size as well as on the geometry of the nozzle and polymer melt viscosity (Comb et al., 1993)³. Higher printing speeds results in the underflow of the polymer melt from the nozzle with poor printing quality as a result. In the a.m. experiments the process parameters and the geometry of the nozzle were kept the same for the drug-free and the drug-loaded filaments. The viscosity of the pure PCL filament and the drug-loaded filaments were almost the same at the printing temperature of 100 ºC (Figure 22). All drug-loaded PCL filaments could be successfully fed into the liquefier and printed without problems at the applied printing temperature.

The XRD, DSC and ATR-IR analysis indicated that there was undissolved indomethacin after the extrusion process in the filaments containing 15% and 30% indomethacin as the printing temperature was far below the melting point of the raw indomethacin.
³ AGARWALA, M., JAMALABAD, V., LANGRANA, N., SAFARI, A., WHALEN, P. and DANFORTH, S., 1996. Structural quality of parts processed by fused deposition. Rapid Prototyping Journal, 2(4), pp. 4-19.

When printing is done at higher temperatures, it takes longer time for the printed polymer to cool down. Sun et al. (2008)⁴ has reported that the thermal history of a material has an impact on the bonding strength between adjacent layers and roads achieved under printing.

The nozzle used in this study was 0.4 mm, and therefore, the shear rate region can be different from the above mentioned. The nozzle length and angle affect the shear rate.

To sum up, the differences in the viscosity profiles between printing materials at two different printing temperatures were responsible for the quality of the frames. Generally the printing temperature should be up to 10% above the melting point of the selected polymer and below the melding point of the contained drug. At temperatures of 165 °C frames with an unacceptably poor quality had been obtained, ia. since the melting point of indomethacin is γ-indomethacin is 158°C and thus below this value. Melting of the drug has also a negative impact on the release kinetic of the final product.

In addition, the problems in adhesion between the subsequent layers and roads of the materials during printing at higher temperature add to that matter. This is in accordance with the results Comb et al. (1993)³ reported about the modelling zone, whereas printing above a threshold temperature the printing quality is poorer than printing at lower temperatures.

Therefore, the T-frames of the current invention were printed at 100 °C, where the best results have been obtained.

The mean weight and the weight variation (SD) of the printed drug-loaded IUS T-frames is dependent on the drug load. The smallest variation was reached in the T-frames containing 5% indomethacin. The 30% drug-containing T-frames had the highest weight variation and the lowest weight. This was due to the fact that there was a higher amount of drug particles present in the polymer melt, which made the printing more difficult, leading to the poorer quality of the printed T-frames with highest drug loading.

The loading of the filaments inside the printer extruder was a problem with both the drug-free and drug-loaded EVA filaments. Despite the fact that the filaments were of the right diameter, the loading process did not always succeed. Most of the elastic EVA grades could not act as a piston to push the melted polymer through the nozzle, and therefore, they were bended or buckled above the liquefier during the loading stage. This was due to too low column strength of the filament. The column strength is a function of the filament diameter, flexural modulus and strength of the filament (Comb et al., 1994)². The diameter of all
⁴ SUN, Q., RIZVI, G.M., BELLEHUMEUR, C.T. and GU, P., 2008. Effect of processing conditions on the bonding quality of FDM polymer filaments. Rapid prototyping Journal, 14(2), pp 72-80
filaments was the same, and equal to 1.75 ± 0.05 mm. The filaments' flexural or tensile strength could not be an issue, since none of the filaments were deformed or broke under the loading procedure.

The flexural modulus shows the tendency for a material to bend. The flexural modulus of the EVA grades and PCL was 7-123 MPa and 411 MPa. The flexural modulus of the EVA filaments was much lower than for the original feedstock PCL. The value decreased with increased VA content of the EVA polymer.

It was found that most of the EVA grades with flexural modulus values between 42 MPa and 123 MPa could be fed into the liquefier. However, EVA 6 with the flexural modulus value of 45 was not fed successfully, which was due to high viscosity.

Besides the column strength, the viscosity of the melt was critical for the loading and printing process to succeed. The force needed to press the melt through the nozzle depends on the pressure drop in the nozzle. The pressure drop depends on the geometry of the print head and the viscosity of the melt. Since the geometry of the print head was the same for all printing experiments, the pressure drop variation depended on the viscosity of the melt. A material with higher viscosity needs more power from the piston acting filament to be extruded through the nozzle. The used EVA grades had melt indexes (MI) varying between 1.1-150 g/10min and 500 g/10min. The MI is a measure of the ease for a melt to flow under pressure, at a defined temperature. The MI increases with increased VA content and decreased molecular weight of EVA polymer. If the MI was too low, the drop pressure was too high for the filament to push the melt through the nozzle. EVA grades that were successfully loaded had MI between 2.8 and 500, but not all of them in that range could be fed because of low flexural modulus value. Too add, the MI values reported in the manufacturer material sheets were measured at 190 °C, which differ from the applied printing temperatures. Rheological tests were not performed to determine the MI at the printing temperature, and therefore, the exact MI values at the printing temperature are not revealed. Not only low MI was a problem in the FFF process with EVA. If the MI was too high, which was the case with the EVA 8 with MI 500 g/10min, the polymer was easily fed (in spite of low value of flexural modulus) but it was extruded as droplets, not as a continuous line, and as a result the printing failed. That was also the case with EVA 11, but the exact MI value was not revealed because it was a blend with 50% of EVA 8.

Besides the material properties, hardware properties, such as the pinch rollers surface and groove depth affect the loading process. Some of the problems with the filament loading process of EVA, was due to slipping between the pinch rollers. The rollers surface and the groove depth have to match with the printing material to prevent slipping.

The printing speeds for EVA varied between 10-40 mm/s. As discussed under the printing of PCL, the parameters that affect the printing speed are the printer extruded roads width and height, printing temperature and nozzle size, but also the geometry of the nozzle and polymer melt viscosity.

In the literature it has been reported that the thermal behavior of PCL in the liquefier differ from other commonly used FFF feedstock, e.g. ABS (Ramanath et al. 2008)⁵. The liquefier length required for PCL to fully melt is much shorter than for ABS. The thermal behavior in the liquefier of the EVA polymer was not determined. The melt behavior of the EVA polymer can differ from PCL, and the required length of the liquefier can be longer than for the original liquefier optimized for PCL. It is possible that EVA needs longer time in the liquefier before it melts and that in turn affect the printing speed. Printing experiments was done at higher temperatures with higher speeds, but the printing result was poorer, due to weaker bonding between layers.

Since the printing temperature was above the melting point of the drug, a printing experiment was done at 135 ºC for the 15%-indomethacin loaded filament, to be able to compare between the dissolution profiles of T-frame containing melted (165 ºC) or crystalline (135 ºC) drug. The 15%-indomethacin had a higher viscosity at 135 ºC, than for 165 ºC due to crystalline indomethacin present in the polymer. The higher viscosity made it impossible to print or even load the filament at 135 ºC due to buckling of the filament. The higher viscosity at 135 ºC increased the liquefier pressure (e.g. pressure drop), and the column strength of the filament was exceeded with buckling as a result.

The unloaded PCL filament was white and opaque after extrusion. The fabricated filaments containing IND turned to be of yellow color. The filament with 5% indomethacin was yellow and translucent. The filament with 15% indomethacin was slightly brighter yellow and opaque. The filament with 30% indomethacin was opaque, but lighter yellow than the 15% filament. It is known that dissolved and amorphous indomethacin has a yellow color. Further investigations, e.g. XRD, DSC and ATR-IR confirmed that the drug had dissolved in the polymer melt to some extent. The different shades of yellow color were due to the fact that there were different amounts of undissolved drug in the filaments.

SEM analysis was performed on approx. 3 months old filaments to get further insight into the morphology of the samples. The surface of the drug-free and up to 15% drug-loaded filaments were smooth, but on the surface of the 30% drug-loaded filaments some cracks
⁵ RAMANATH H.S., CHUA C.K., LEONG K.F, SHAH K.D., 2008. Melt flow behaviour of poly-E-caprolactone in fused deposition modelling. Journal of material science. Materials in medicine, 19(7) pp. 2541-2550
could be seen. The cross-sections of the drug-loaded filaments the surface was not as smooth as of the drug-free filament, which was due to small drug particles. The cross-section of the extruded 30% drug-loaded filament showed a more uneven surface than the others.

The HME extrusion process of the EVA grades was carried out at 105-120 °C, depending on the melting point and viscosity of different grades. EVA grades with a lower VA content has a higher molecular weight, which increases the polymer melt viscosity (Almeida et al., 2011)⁶.

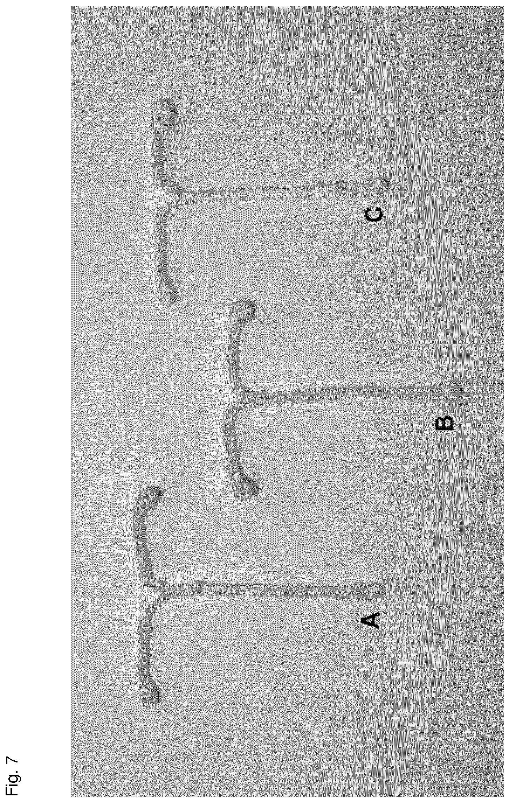

The filaments of all the extruded drug-free EVA grades were translucent. When the EVA grades (EVA 3 and EVA 5) were extruded with indomethacin, the extruded drug loaded filaments were opaque and white. The filament containing 15% was a bit whiter than the filament containing 5% indomethacin (Figure 7, left). Since the extrusion temperature was below the melting point of the drug, the drug had not melted. The color indicates also that the indomethacin had not dissolved in the melted polymer, since the filaments had not turned yellow. From literature it can be concluded that EVA 5 with a VA content of 16% has a solubility parameter between 16.33-17.4 MPa^{1/2}. The solubility parameter for other grades of EVA is between 16.33-18.38 MPa^{1/2}.

The drug release profiles from the printed devices of the invention were faster than from the corresponding filaments due to the difference in the degree of the crystallinity of the incorporated drug and the geometry of other products.

### Example 4 (in vitro drug release)

The release experiments were started by selecting the media in which the release testing will be done and by making standard curves of indomethacin in those media. The possible release media were purified water, 0.9% NaCl and 1 % (2-Hydroxypropyl)-β-cyclodextrin.

### Example 4a)

### in vitro drug release from PCL filaments and 3D printed T-frames (subsequently also named prototypes)

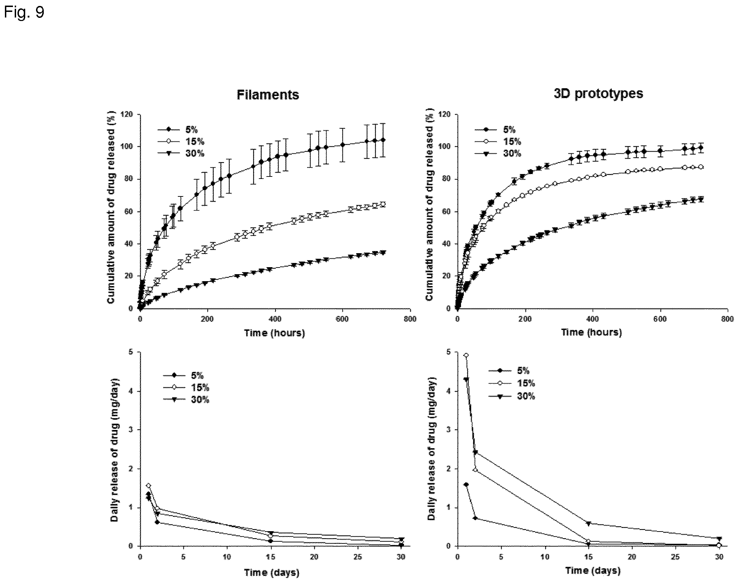

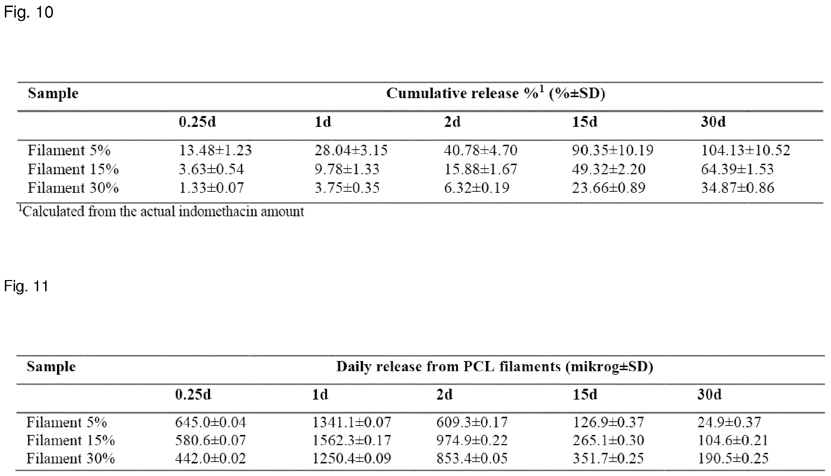

In the tables (figures 10 and 11) and figure 9 (left) the cumulative percentage and the daily mean release data of IND from PCL filaments (1-3 weeks old) over a period of
⁶ ALMEIDA, A., POSSEMIERS, S., BOONE, M.N., DE BEER, T., QUINTEN, T., VAN HOOREBEKE, L., REMON, J.P., VERVAET, C., 2011. Ethylene vinyl acetate as matrix for oral sustained release dosage forms produced via hot-melt extrusion. European Journal of Pharmaceutics and Biopharmaceutics, 77(2), pp. 297-305
30 days *in vitro* release test under sink conditions are presented. The filament containing 5% indomethacin showed an initial burst release phase. After the initial fast release the drug release rate gradually slowed down followed by a sustained release phase. The filaments containing 15% and 30% showed a lower initial burst release. The initial fast release was due to immediate dissolution of the drug located on or near the surface of the filament. After the initial phase, the drug was released slowly by diffusion of drug molecules from the interior of the polymer matrix. The overall drug release percentage was highest for the filament containing 5% indomethacin and lowest for the filament with 30% indomethacin after 30 day release. Based on XRD, DSC and ATR-IR analysis the drug had completely or almost completely dissolved under extrusion only in the filament containing 5 %. In both the filaments containing 15 % and 30 % indomethacin, the drug was at least partially in its crystalline state. The dissolution rate of amorphous indomethacin or dissolved indomethacin is faster than the crystalline counterpart, and therefore, the release percentage of the drug is higher from the filaments containing almost or near almost dissolved indomethacin (5%).

As expected, the overall daily release amount of the drug decreased faster for the filament containing 5% indomethacin than for the two other filaments. The filament containing 15% indomethacin released higher amount of the drug during the first days than the filament containing 30%. Evidently, the highest amount of the molecularly dispersed drug was present on the surface of the filament containing 15% indomethacin. After a few days, the slowest decrease in the drug amount was observed for the filament with highest drug loading as the slow diffusion from the interior to the exterior of all filaments became the predominant release mechanism.

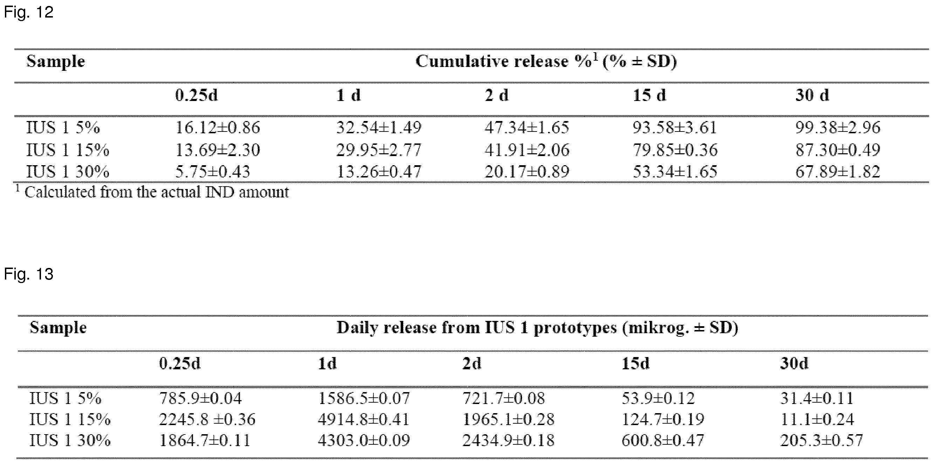

In the tables (figures 12 and 13) and figure 9 (right) the cumulative release and the daily release data of IND from the 3D printed PCL IUS 1 implants (1-2 weeks old) over a period of 30 days is presented. All three prototypes showed an initial burst release phase. The first burst release phase was followed by slow diffusion of the drug from interior to exterior through the voids, remained after already released drug molecules/crystals. The initial burst release was lower for the prototypes with the highest drug loading. After the initial fast release a sustained drug release phase was monitored. The drug release was fastest for the prototype containing 5% indomethacin, and slowest for the prototype with 30% indomethacin. The release profiles from the prototypes with 5 % and 15 % indomethacin were closer to each other than in the case of the corresponding filaments. It can be explained with the fact that the drug in those prototypes was mainly present in the molecularly dispersed state, whereas in the 15% drug-loaded filament contained the drug mostly in the crystalline form. The geometry of the extruded filaments and the printed prototypes differ, and therefore, the release rate cannot be compared directly. In Figure 7 pictures of all drug-loaded IUS 1 after drug release is presented.

### Example 4b)

### in vitro drug release from EVA 5 filaments and 3D printed T-frames (subsequently also named prototypes)

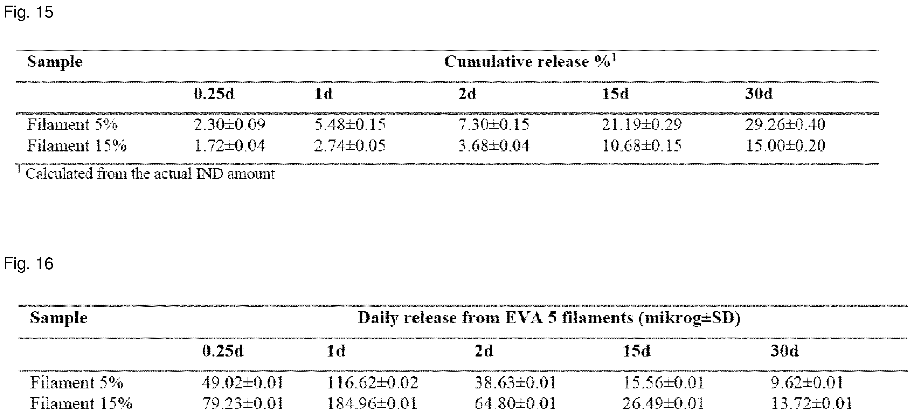

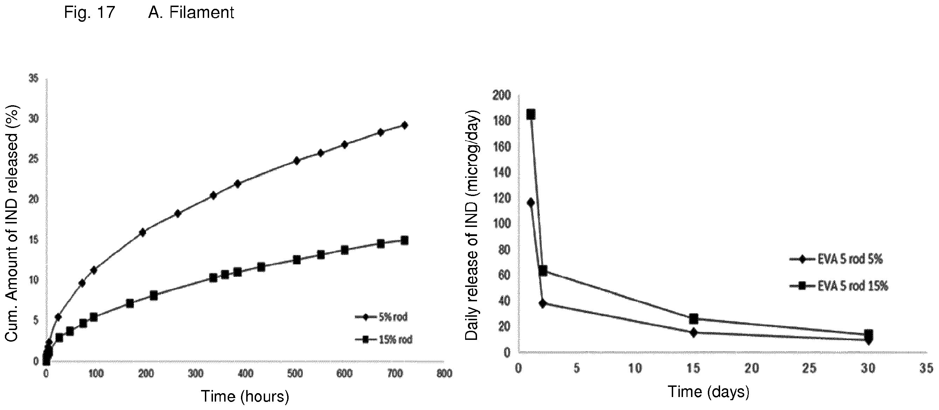

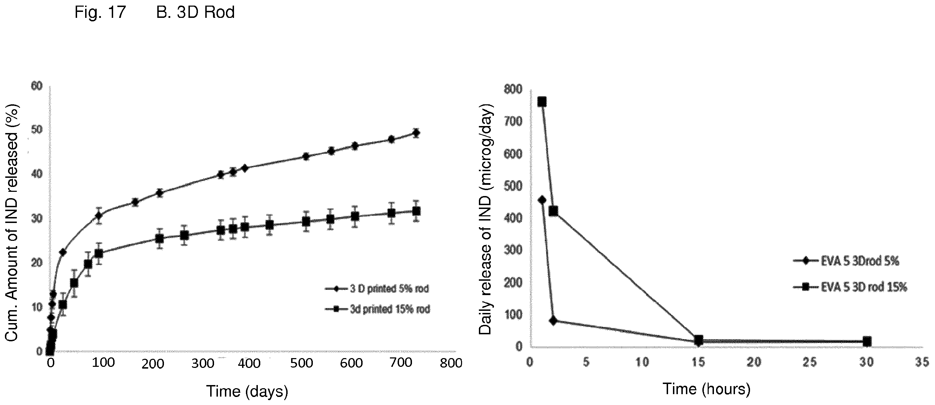

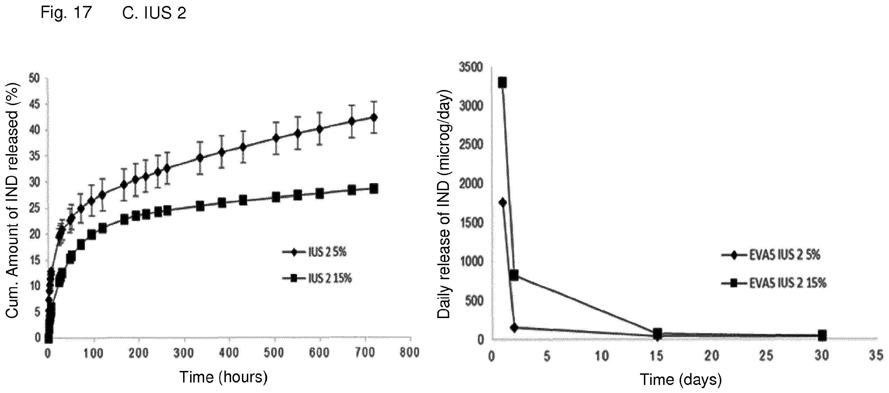

The cumulative and daily release of indomethacin from the EVA 5 filaments is presented in tables (figures 15 and 16) and in figure 17 (A filament). The cumulative percentage drug release after 30 days was higher from the filament containing 5% than from the one containing 15%. This is in accordance with previous results presented in literature (Andersson et al., 2011)⁷, with an EVA grade of VA-content of 18% with etonogestrel as a model drug. In a study with an EVA containing 40% VA with a crystalline freely water soluble drug, the release rate was faster from devices with higher drug loadings (Almeida et al., 2011)⁸. Almeida et al. (2011) reported that the release rate from EVA is a combination of different parameters, such as drug crystallinity, polymer crystallinity, drug loading and extrusion temperature. In addition, drug solubility in the release medium plays an important role and affects the release rate of the drug from the polymer at some extent. SEM images of the surfaces of 5 % drug-loaded filaments before and after dissolution have been measured. After drug release the surface is more porous because of disappearance of drug particles.

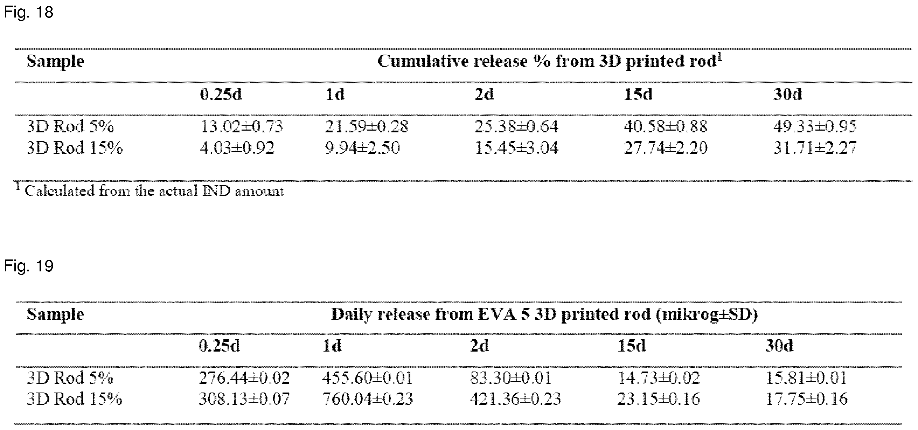

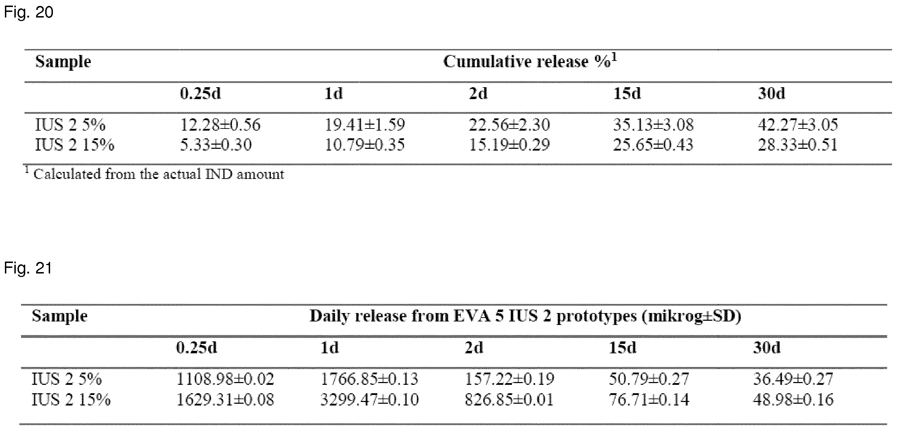

The drug release from the 3D printed rods and IUS 2 prototypes containing 5% indomethacin was faster than the drug release from the counterparts containing 15% indomethacin [Figure 17 B 3D Rod; and C IUS 2 and Tables (figures 18-21)]. Both
⁷ ANDERSON, K., et al. Controlled release of Active Pharmaceutical Ingredients from Ethylene Vinyl Acetate Copolymers, Celanese White Paper, 2011
⁸ ALMEIDA, A., POSSEMIERS, S., BOONE, M.N., DE BEER, T., QUINTEN, T., VAN HOOREBEKE, L., REMON, J.P., VERVAET, C., 2011. Ethylene vinyl acetate as matrix for oral sustained release dosage forms produced via hot-melt extrusion. European Journal of Pharmaceutics and Biopharmaceutics, 77(2), pp. 297-305
exhibits a burst release during the first days. The release rates are higher than those for the extruded filaments, which is due to the fact that the printing temperature was above the melting point of the drug. According to the XRD, DSC and ATR-IR analysis most of the drug had melted and/or dissolved during the printing, which made the drug release from the printed rods faster than from the extruded counterparts.

In conclusion, the drug release from the printed devices depended on the geometry of the devices, the matrix polymer and the degree of the crystallinity of the incorporated drug. The drug release rate was slower for the devices with a bigger device diameter. The drug release rate from the EVA polymer was slower than for the PCL polymer. The cumulative percentage drug release was slower from the devices with higher drug loading than from those with lower drug loading, this was due to the fact that in the devices with higher drug loading there were more crystalline drug that in those with lower drug loading.

### Description of Figures

- Figure 1.: Manufacturing process of the IUS and IVR devices and the characterization methods used in this study are schematically presented. The drug content analysis and the viscosity measurements were done only for the filaments

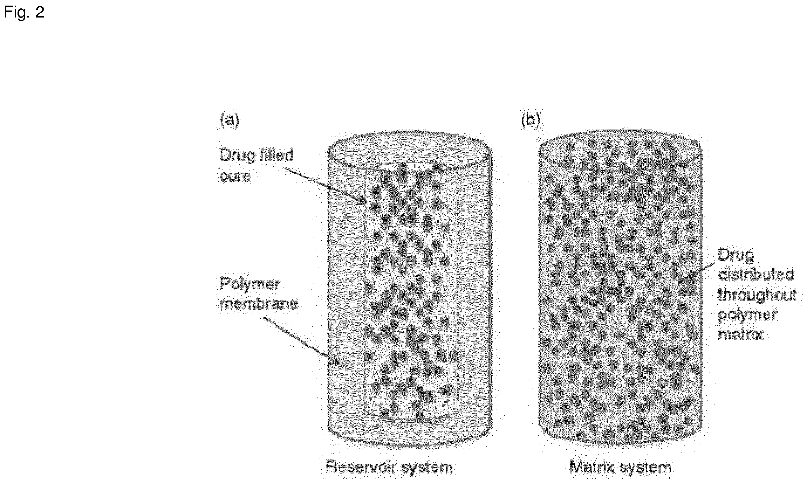

- Figure 2.: Schematic representation of a (a) reservoir- and a (b) matrix or monolithic drug delivery system (Solorio et al., 2014)⁹
⁹ SOLORIO, L., CARLSON, A., ZHOU, H. and EXNER, A.A., 2014. Implantable drug delivery systems in Bader, A.R. and Putnam D.A. (ED); Engineering polymer systems for drug delivery, John Wiley & sons, Inc., 2014
- Figure 3: Shows polymer properties for EVA polymers with various VA content and PCL (CAPA™6500; Perstorp) in dependency of the process parameters in the hot melt extrusion process. PCL The ratio of EVA / VA in the polymer mixture is given in weight percent. It has been found that with EVA 5 (16 wt/% VA) and EVA 7 (18 wt% VA) the best results and the best printability were obtained. Material properties of PCL, EVA 3 and EVA 5 had been tested also for the drug drug loaded (indomethacin) polymer
- Figure 4:: Screenshots of the T-frames in *Rhinoceros 5.0* software

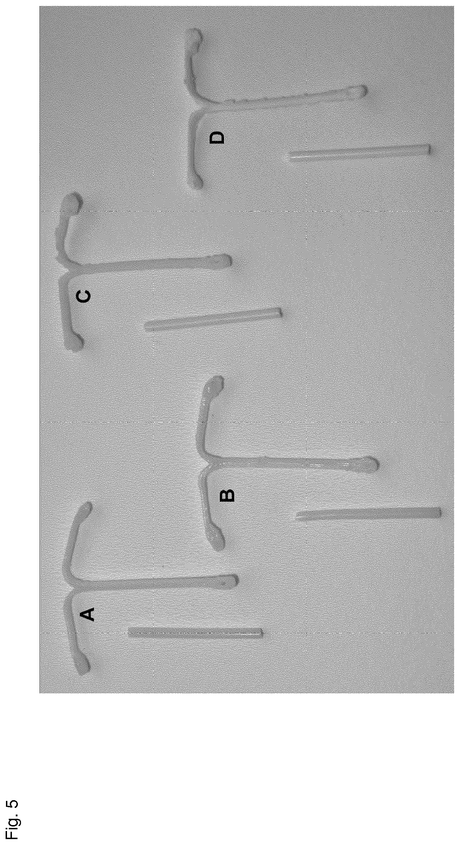

- Figure 5:: Printed T-frames and filaments of PCL: (A) Pure PCL, (B) 5% Indomethacin, (C) 15% Indomethacin and (D) 30% Indomethacin

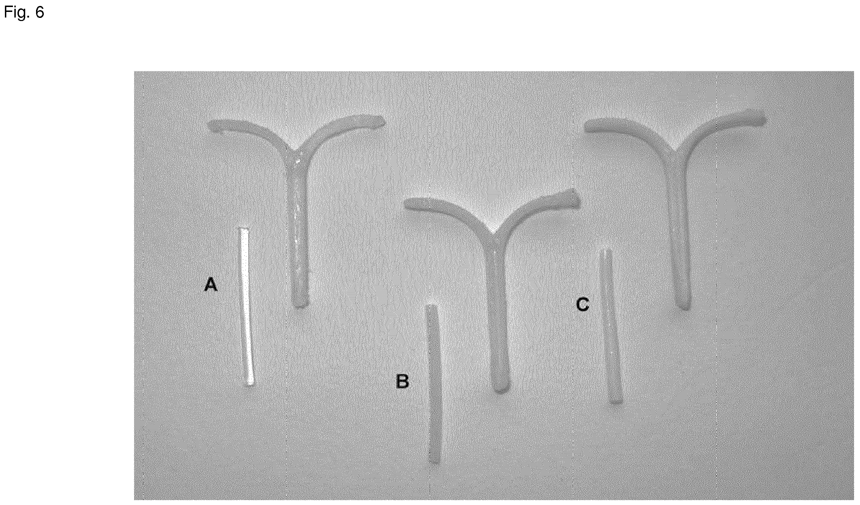

- Figure 6:: Filaments and printed T-frames of EVA 5: (A) drug-free, (B) 5% Indomethacin-containing and (C) 15% Indomethacin-containing filaments and IUS 2 T-frames
- Figure 7:: 3D printed IUS1 T-frames with: (A) 5%, (B) 15% and (C) 30% Indomethacin loading after release tests
- Figure 8:: An illustration of a typical FDM™/FFF extruder (Turner et al., 2014)¹⁰
¹⁰ TURNER, B., STRONG, R. and GOLD, S.A., 2014. A review of melt extrusion additive manufacturing processes: I. Process design and modeling. Rapid Prototyping Journal, 2014, 20(3), pp. 192-204
- Figure 9:: Cumulative percentage release (top) and daily release (bottom) of indomethacin from PCL filaments and 3D prototypes
- Figure 10:: Cumulative percentage release of drug from PCL filaments with different drug loading content (n=3)
- Figure 11:: Daily release of indomethacin from PCL filaments
- Figure 12:: Cumulative percentage indomethacin release from PCL IUS 1 prototypes (n=3)
- Figure 13 :: Daily drug release from the PCL IUS 1 prototypes (n=3)

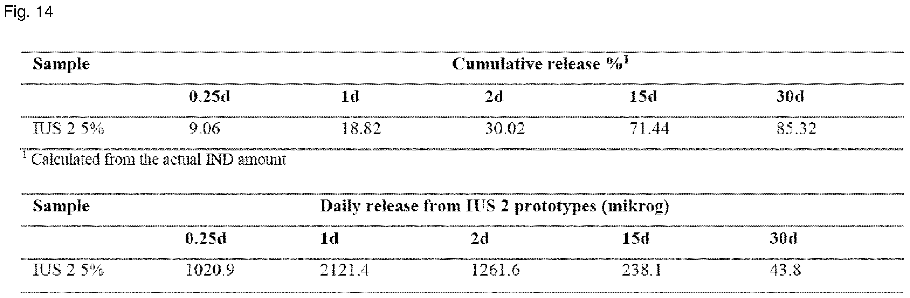

- Figure 14 :: Cumulative percentage release and daily release from IUS 2 prototypes (n=1)
- Figure 15 :: Cumulative percentage release from EVA 5 filaments (n=3).
- Figure 16 :: Daily release from EVA 5 filaments (n=3)
- Figure 17 :: The cumulative and daily release from (A) EVA 5 filaments, (B) EVA 5 3D printed rods and (C) EVA 5 3D printed IUS 2 prototypes
- Figure 18:: Cumulative percentage release from 3D printed EVA 5 rods (n=3).
- Figure 19:: Daily release from 3D printed EVA 5 rods (n=3)
- Figure 20:: Cumulative percentage release from EVA IUS 2 prototypes (n=3)
- Figure 21:: Daily release from EVA 5 IUS 2 prototypes (n=3)
- Figure 22:: The viscosity versus shear rate of PCL filaments, (··■··) pure PCL filament, (◆) PCL 5% IND, (■) PCL15% IND, (▲) PCL 30% IND at 100 °C and (●) PCL 30% IND at 165°C

## Claims

1. An intrauterine delivery system comprising Indomethacin and Levonorgestrel, **characterized in that** the Indomethacin is contained in the frame material and that levonorgestrel is contained in a silicon based reservoir attached to the frame, wherein the frame consist of ethinylvinylacetate copolymer with 16 % vinylacetate content (EVA 5).

2. A method for manufacturing of a frame of an intrauterine delivery system comprising Indomethacin and Levonorgestrel, wherein the Indomethacin is contained in the frame and levonorgestrel is contained in a silicon based reservoir attached to the frame , **characterized in, that** a mixture of a ethinylvinylacetate copolymer with 16 % vinylacetate content (EVA 5) and the Indomethacin are treated under the condition of 3-D printing methods.

3. A method of claim 2, wherein the intrauterine delivery system for the controlled release of progestogen or a drug having a progestogenic activity over a prolonged period of time and at a level required for contraception, and the system comprises a body construction and at least one silicon based reservoir comprising a core and optionally a membrane encasing the core, the core and membrane essentially consisting of a same or different polymer composition, **characterized in that** said intrauterine delivery system comprises levonorgestrel as a progestogen or a drug having progestogenic activity.

4. A method according to claim 2, wherein the ranges of indomethacin are 5 - 40 wt-%.

5. A method according to claim 4, wherein the amounts of indomethacin are 5 or 15 or 30 wt-%.

6. A method according to claim 4, or 5 wherein the indomethacin is amorphous or α-indomethacin.

## Patentansprüche

1. Intrauterines Verabreichungssystem, welches Indomethacin und Levonorgestrel umfasst, **dadurch gekennzeichnet, dass** das Indomethacin im Rahmenmaterial enthalten ist und das Levonorgestrel in einem am Rahmen befestigten Reservoir auf Siliciumbasis enthalten ist, wobei der Rahmen aus Ethinyl-Vinylacetat-Copolymer mit einem Vinylacetatgehalt von 16% (EVA 5) besteht.

2. Verfahren zur Herstellung eines Rahmens für ein Indomethacin und Levonorgestrel umfassendes intrauterines Verabreichungssystem, wobei das Indomethacin im Rahmen enthalten ist und das Levonorgestrel in einem am Rahmen befestigten Reservoir auf Siliciumbasis enthalten ist, **dadurch gekennzeichnet, dass** eine Mischung eines Ethinyl-Vinylacetat-Copolymers mit einem Vinylacetatgehalt von 16% (EVA 5) und dem Indomethacin unter 3D-Druck-Verfahrensbedingungen behandelt wird.

3. Verfahren nach Anspruch 2, wobei das intrauterine Verabreichungssystem für die gesteuerte Freisetzung von Progestogen oder einem Arzneimittel mit progestogener Wirkung über einen längeren Zeitraum und in einer für die Empfängnisverhütung erforderlichen Konzentration, und das System eine Körperkonstruktion und mindestens ein Reservoir auf Siliciumbasis umfassend einen Kern und gegebenenfalls eine den Kern einfassende Membran umfasst, wobei der Kern und die Membran im Wesentlichen aus einer gleichen oder verschiedenen Polymerzusammensetzung bestehen, **dadurch gekennzeichnet, dass** das intrauterine Verabreichungssystem Levonorgestrel als Progestogen bzw. Arzneimittel mit progestogener Wirkung umfasst.

4. Verfahren nach Anspruch 2, wobei die Menge an Indomethacin im Bereich von 5-40 Gew.-% liegt.

5. Verfahren nach Anspruch 4, wobei die Mengen an Indomethacin 5 oder 15 oder 30 Gew.-% betragen.

6. Verfahren nach Anspruch 4 oder 5, wobei es sich bei dem Indomethacin um amorphes Indomethacin oder α-Indomethacin handelt.

## Revendications

1. Système d'administration intra-utérine comprenant de l'indométacine et du lévonorgestrel, **caractérisé en ce que** l'indométacine est contenue dans le matériau du cadre et que le lévonorgestrel est contenu dans un réservoir à base de silicium relié au cadre, le cadre étant constitué de copolymère d'éthinyl-acétate de vinyle présentant une teneur en acétate de vinyle de 16% (EVA-5).

2. Procédé pour la fabrication d'un cadre d'un système d'administration intra-utérine comprenant de l'indométacine et du lévonorgestrel, l'indométacine étant contenue dans le cadre et le lévonorgestrel étant contenu dans un réservoir à base de silicium relié au cadre, **caractérisé en ce qu'**un mélange d'un copolymère d'éthinyl-acétate de vinyle présentant une teneur en acétate de vinyle de 16% (EVA-5) et de l'indométacine est traité dans des conditions de procédés d'impression 3-D.

3. Procédé selon la revendication 2, le système d'administration intra-utérine pour la libération contrôlée de progestatif ou d'un médicament possédant une activité progestative pendant une durée prolongée et à un niveau requis pour une contraception et le système comprenant une construction de corps et au moins un réservoir à base de silicium comprenant un noyau et éventuellement une membrane enveloppant le noyau, le noyau et la membrane étant essentiellement constitués d'une composition polymère identique ou différente, **caractérisé en ce que** ledit système d'administration intra-utérine comprend du lévonorgestrel en tant qu'un progestatif ou un médicament possédant une activité progestative.

4. Procédé selon la revendication 2, les plages d'indométacine étant de 5-40% en poids.

5. Procédé selon la revendication 4, les quantités d'indométacine étant de 5 ou 15 ou 30% en poids.

6. Procédé selon la revendication 4 ou 5, l'indométacine étant de l'indométacine amorphe ou α.
